# EUROPEAN PATENT SPECIFICATION

(11) **EP 0 378 684 B1**
(45) Date of publication and mention of the grant of the patent: **09.03.1994**
(21) Application number: 88908333.3
(22) Date of filing: 16.09.1988
(51) Int. Cl.: B01D 61/00, B01D 39/16, A61K 35/14, A61M 1/16, A61M 1/34

(54) **LEUCOCYTE-SEPARATING PROCESS**
LEUKOZYTEN-TRENNVERFAHREN
PROCEDE DE SEPARATION DE LEUCOCYTES

(30) Priority: 18.09.1987 JP 232066/87
(43) Date of publication of application: 25.07.1990
(73) Proprietor: TERUMO KABUSHIKI KAISHA, Tokyo 151 (JP)
(72) Inventor: NAOI, Keiji, Shibuya-ku Tokyo 151 (JP)
(74) Representative: Gillard, Marie-Louise
(86) International application number: JP8800942
(87) International publication number: WO8902304

(56) References cited:
- GB-A- 2 024 827
- JP-A- 5 446 811
- JP-A- 5 724 605
- JP-A-55 136 955
- JP-A-58 180 425
- JP-A-61 226 056
- JP-B- 6 139 060

## Description

### [Technical Field]

This invention relates to a process for separating leukocytes from blood containing leukocytes and erythrocytes. More particularly it relates to the separation of leukocytes by using a filter which exhibits a stable ability to capture leukocytes and has no possibility of contamination by foreign matter.

### [Background Art]

It is long since the form of blood transfusion began to change from the conventional whole blood transfusion to the blood component transfusion which consists in transfusing only the particular component of blood to be required by a given patient. The task set for this blood component transfusion resides in the search for a way of heightening the purity with which each of blood components is to be fractionated is to be obtained.

It has been customary for the blood from donors to be centrifugally separated into concentrated red cells (CRC), platelet concentrate (PC), and platelet poor plasma (PPP). The concentrated red cells thus separated from the blood are prepared in the form of a componental drug of red cells and used extensively for the blood component transfusion to patients who are in need of red componental drug of red cells. The view that the concentrated red cells contain leukocytes and platelets in large proportions and constitute the so-called total component blood has been finding popular recognition. The fact that the transfusion of these concentrated red cells, therefore, inevitably compels a patient who needs only red blood cells to admit involuntarily large volumes of leukocytes and platelets has been arousing much anxiety about the desirability of the transfusion. The leukocytes and platelets which are contained in the red blood fraction such as these concentrated red cells must be removed to the fullest possible extent even for the sake of precluding otherwise possible occurrence of post transfusion reaction. To date, various methods or devices have been invented with a view to meeting this purpose.

For the elevation of the purity of a red cell drug, the method of centrifugal separation which makes use of variation in specific gravity between different species of blood cells, the method which adopts a sequestrating material operating by virtue of the phenomenon of adhesion of blood cells, and the method which effects separation of leukocytes by the use of a red cell agglutination agent have been put to use.

Among the other methods mentioned above, the method which adopts the sequestrating material has found particularly popular acceptance in the light of high efficiency in the removal of leukocytes and high convenience of procedure. As the sequestrating agent, such fibers as natural cellulose, polyester, polyamide, polyacrylonitrile, and glass fibers which have extremely small diameters are used in most cases as packed in their unmodified form or as further processed into non-woven fabrics, for example.

When the fibers are to be packed in their unmodified form in a column for the purpose of the method mentioned above, since it is difficult to have the fibers uniformly in the column, the preparation of the packed column calls for much time and labor. Further, depending on the manner in which the fibers are packed in the column, there is a fair possibility that the packed column develops the phenomenon of channeling while in operation. If the packing density of fibers is increased so as to ensure through capture of leukocytes, the time for filtration is notably elongated. Further, since the individual fibers so packed in an increased density are not allowed to be amply intertwined, the possibility ensues that they may become loose and flow out of the column while the column is in service. When the fibers are used as additionally processed into a non-woven fabric, for example, though the problem mentioned above does not occur readily, the disadvantage persists that the blood cells captured on the non-woven fabric tend to clog the fabric.

A sequestrating material capable of manifesting an ample and stable performance as a filter for the removal of leukocytes remains yet to be developed. Such is the true state of affairs.

An object of this invention, therefore, is to provide a novel process for separating leukocytes from blood containing leukocytes and erythrocytes. Another object of this invention is to provide a process wherein a filter is used, which possesses a high and stable ability to capture leukocytes and is capable of efficiently separating leukocytes from blood. A further object of this invention is to provide a process wherein the filter employed is capable of safely effecting the removal of leukocytes without entailing the otherwise possible leakage of foreign matter. Yet another object of this invention is to provide a process wherein the filter used for the removal of leukocytes can be produced by a simple procedure without entailing any noticeable dispersion of product quality.

### [Disclosure of Invention]

The objects described above are accomplished by a process for separating leukocytes from blood containing leukocytes and erythrocytes in accordance with independent claim 1.

Dependent claims 2 to 4 represent preferred embodiments of the process of the invention.

It is to be noted that a filter in the form of a porous polyvynil formal sponge is already known from SE-A-441 894 (& JP-A- 59 180425) as useful for filtering preserved blood, for the purpose of removing fine coagulated blood.

### [Brief Description of Drawings]

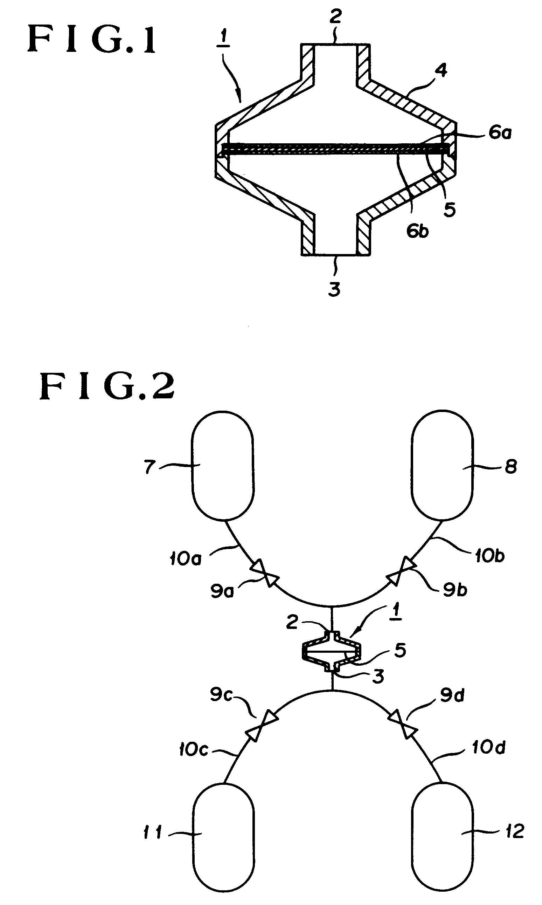

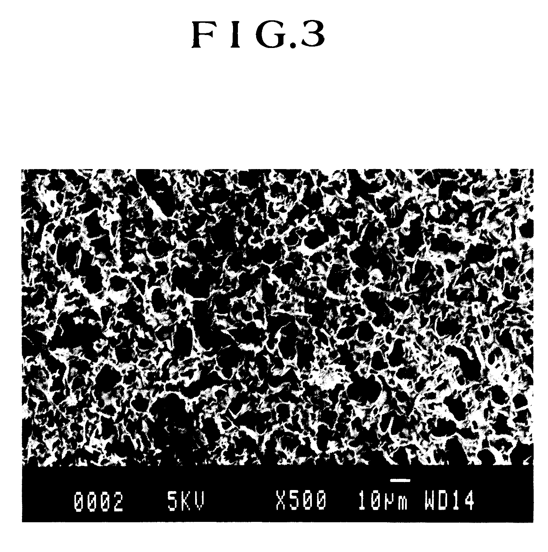

Fig. 1 is a cross section of a typical filter for the separation of leukocytes as used in one embodiment of the process of the present invention. Fig. 2 is a circuit diagram illustrating a circuit incorporating the typical filter mentioned above and used for the treatment of blood. Fig. 3 is an electron micrograph illustrating a typical microstructure of a porous polyvinyl formal article used in the filter of the process of this invention for the separation of leukocytes.

### [Best Mode for Carrying out the Invention]

The filter employed in the process of this invention for the removal of leukocytes is characterized by being made of a porous polyvinyl formal article of a three-dimensionally reticular continuous texture possessing continuous open pores 5 to 30 µm in average diameter. When the porous polyvinyl formal article having as a matrix thereof a three-dimensionally reticular continuous texture possessing pores of a diameter falling in a prescribed range as described above is used for treating a leukocyte suspension such as the blood or the concentrated red cells, the leukocytes contained in the leukocyte suspension are adsorbed and captured quite efficiently by the inner surfaces of pores while the suspension is being passed through complicate flow paths formed in the matrix of the porous article by the continuous open pores. The flow paths of the filter are formed by the three-dimensionally reticula continuous texture of the porous article, namely the continuous open pores formed in the matrix of the porous article. Since the flow paths of the filter are formed while the porous article is in process of production, the procedure to be involved in the manufacture of a filter for the removal of leukocytes by the use of the porous article is extremely simple and the possible dispersion of product quality is insignificant. Further, since the matrix of the porous article is a continuous texture, it is stable and is substantially free from such drawbacks as leakage of foreign matter from the porous article or channeling of flow path during the operation of the filter.

Now, the present invention will be described in detail below with reference to embodiments thereof.

The filter employed in the process of this invention for the removal of leukocytes is made of a porous polyvinyl formal article of a three dimensionally reticular continuous texture possessing such continuous open pores as illustrated in Fig. 3, for example.

In the porous polyvinyl formal article possessing such a texture as described above, the continuous open pores must possess an average diameter in the range of 5 to 30 µm, more desirably 8 to 30 µm, and most desirably 10 to 25 µm. If the average pore diameter is less than 5 µm, even the red cells which are contained in the leukocyte suspension such as the blood or the concentrated red cells under treatment are unintentionally captured during the course of the removal of leukocytes, with the result that the ratio of recovery of red cells is lowered and the porous article is possibly clogged in consequence of the capture of an overwhelming large number of red cells. Conversely if the average pores diameter exceeds 30 µm, the frequency of contact of the porous article with the leukocyte suspension under treatment is lowered possibly to the extent of lowering the ratio of capture of leukocytes. The term "average pore diameter" as used in this specification refers to what is obtained by cutting a cross section randomly across a given porous article, measuring diameters of all the pores exposed throughout the entire area of the cross section, grouping these pores by diameter, reducing the pores in the largest of the groups into circles, and calculating the average diameter of the circles. Specifically, the pores distributed in a given cross section across the porous article are widely varied in shape and in diameter as well. The individual pores are reduced into circles of equal surface area and the results of the reduction are plotted in a graph, using the lateral axis thereof as the scale for diameter and the vertical axis as the scale for number of pores. Generally, the data describe a curve closely approximating the normal distribution. The data are desired to be obtained with respect to at least 1,000 pores randomly selected in the cross section. The diameter falling at the peak of the curve is reported as the average pore diameter in the specification. When the filter made of this porous article is used for screening a liquid containing particles varying in size, therefore, those of the particles having diameters larger than the average pores diameter are passed through the filter only with difficulty. The term is not meant to express that particles of larger diameters are never passed through the filter.

Though the porosity of the porous polyvinyl formal article hinges on such factors as average pore diameter, it is desired to be approximately in the range of 75 to 95%, preferably 80 to 90%. This range of the porosity is significant in respect that the filter is capable of effecting the removal of leukocytes with ample quickness when the porosity is not less than 75% and the filter enjoys ample strength when the porosity is not more than 95%. The thickness of the porous polyvinyl formal article may be influenced by such factors as average pore diameter, porosity, and microfine structure of the three dimensionally reticula continuous texture of matrix. It is generally in the range of 0.5 to 5.0 mm, preferably 0.5 to 3.0 mm. The reason for this range of thickness is that the filter enjoys ample strength when the thickness is not less than 0.5 mm and the depth of the filtration layer in the filter is not very large and the possibility of the filter being clogged with coarse particles is small when the thickness is not more than 5.0 mm.

The porous polyvinyl formal article employed in the process of the present invention may be produced by virtually any method, on the sole condition that the method adopted is capable of imparting the specified texture to the produced porous article. The solution method which comprises preparing an aqueous polyvinyl alcohol solution containing a pore-forming agent selected from among amylose-containing polysaccharides such as starch and dextrin, derivatives thereof, acid-proof anionic surfactants, and nonionic surfactants and acetalizing the aqueous polyvinyl alcohol solution with formaldehyde through the agency of an acid catalyst optionally in the presence of such an inorganic salt as sodium sulfate, sodium chloride, ammonium sulfate, ammonium chloride, potassium sulfate, or sodium iodide (Japanese Patent Publications SHO 47(1972)-46,455 and SHO 48(1973)-20,019) proves to be particularly desirable among other methods because the porous polyvinyl formal article to be produced thereby acquires a mechanically stable texture for its large porosity.

Fig. 1 is a cross section illustrating a typical filter for the separation of leukocytes used in one embodiment of the process of the present invention. In the present embodiment, a filter 1 for the separation of leukocytes comprises a housing 4 provided with a blood inlet 2 and a blood outlet 3 and a porous polyvinyl formal article 5 possessing the aforementioned texture and disposed across the inner space of the housing 4. In the leukocyte separation filter 1 of this construction, optionally for the purpose of retaining the porous polyvinyl formal article 5 in place inside the housing 4, liquid-pervious supporting members 6a, 6b may be disposed contiguously to the opposite sides of the porous polyvinyl formal article 5 so as to have the porous polyvinyl formal article 5 nipped between the supporting members 6a, 6b.

This leukocyte separation filter 1 is actually put to use as incorporated in a circuit such as is illustrated in Fig. 2. In the circuit of Fig. 2, a blood bag 7 containing the blood to be treated and a physiological saline solution bag 8 containing physiological saline solution are positioned at a level above the leukocyte separation filter 1 and are made to communicate with the blood inlet 2 of the leukocyte separation filter 1 respectively by means of liquid tubes 10a, 10b which are provided with clamps 9a, 9b. Below the leukocyte separation filter 1 are disposed a physiological saline solution recovery bag 11 and a blood recovery bag 12 for receiving the treated blood. These recovery bags are made to communicate with the blood outlet 3 of the leukocyte separation filter 1 respectively by means of liquid tubes 10c, 10d which are provided with clamps 9c 9d. The operation of the circuit for the separation of leukocytes is started by allowing the physiological saline solution to flow from the physiological saline solution bag 8 to the leukocyte separation filter 1 with the clamps 9b, 9c kept open and the clamp 9a, 9d kept closed thereby priming the interior of the leukocyte separation filter 1. The portion of the physiological saline solution used for the priming is recovered in the physiological saline solution recovery bag 11. After the priming is completed, the blood is allowed to flow from the blood bag 7 to the leukocyte separation filter 1 with the clamps 9b, 9c kept closed and the clamps 9a, 9d kept open. Inside the leukocyte separation filter 1, while the blood is passing the porous polyvinyl formal article 5 possessing the aforementioned texture, this porous polyvinyl formal article 5 captures leukocytes by adsorption. Thus, the blood departing from the porous article 5 is free from leukocytes. The blood thus freed from leukocytes is recovered in the blood recovery bag 12 with which the filter 1 communicates. After the blood bag 7 has been completely emptied of the blood, for the recovery of the portion of blood remaining inside the leukocyte separation filter 1, the flow of the physiological saline solution into the leukocyte separation filter 1 is started opening the clamp 9b after the clamp 9a is closed so as to expel the residual blood from within the leukocyte separation filter 1 and recover it in the blood recovery bag 12. At the time that the recovery of the blood is substantially completed, the clamp 9d is closed and the clamp 9c is opened to recover in the physiological saline solution recovery bag 11 the portion of physiological saline solution which has been used for the recovery of blood. This step completes the operation of the cycle for the separation of leukocytes.

Now, the present invention will be described more specifically below with reference to working examples.

### Example 1

A polyvinyl formal sponge possessing an average pore diameter of 8 µm and a porosity of 86% (produced by Kanebo Ltd. and marketed under product code of "A-3140") was trimmed to a thickness of 1 mm and a cross-sectional area of 100 cm² and used to produce a leukocyte separation filter constructed as illustrated in Fig. 1. This leukocyte separation filter was incorporated in a circuit configurated as illustrated in Fig. 2. Through the filter, 400 cc of CPD-added concentrated human red cells (CRC) adjusted in advance to a hematocrit value of 50% by addition of physiological saline solution was passed. The time required for this treatment was 6 minutes. The cell numbers in the CRC before and after the treatment were measured with an automatic blood cell counter (produced by Ortho-Diagnostic Systems Co. and marketed under product code of "ELT-8"). Then, the absolute amounts of blood components were calculated based on the volumes of liquid. As the result, the ratio of removal of leukocytes was found to be 100% and that of recovery of red cells to be 96% and that of removal of platelets to be 82%.

### Example 2

As polyvinyl formal sponge possessing an average pore diameter of 30 µm and a porosity of 91% (produced by Kanebo Ltd. and marketed under product code of "A-3160") was trimmed to a thickness of 4 mm and a cross-sectional area of 100 cm² and used to produce a leukocyte separation filter constructed as illustrated in Fig. 1. This filter was incorporated in a circuit configurated as illustrated in Fig. 2. Through this filter, 400 cc of CPD-added concentrated human red cells (CRC) adjusted in advance to hematocrit value of 50% by addition of physiological saline solution was passed. The time required for this treatment was 4 minutes. By the same analysis as in Example 1, the ratio of removal of leukocytes was found to be 98%, that of recovery of red cells to be 95%, and that of removal of platelets to be 74%.

### Control 1

A porous article of polyurethane resin possessing an average pore diameter of 30 µm and a porosity of 75% (produced by Toyo Polymer K.K. and marketed under trademark designation of "RUBYCELL") was trimmed to a thickness of 4 mm and a cross-sectional area of 100 cm² and used to produce a leukocyte separation filter similar to that of Example 1. This filter was incorporated in a circuit configurated as illustrated in Fig. 2. Through this filter, 400 cc of CPD-added concentrated human red cells (CRC) adjusted in advance to a hematocrit value of 50% by addition of physiological saline solution was passed. The time required for this treatment was 5 minutes. By the same analysis as in Example 1, the ratio of removal of leukocytes was found to be 35.6%, that of recovery of red cells to be 95%, and that of removal of platelets to be 35%.

### Control 2

A porous article of polyurethane resin possessing an average pore diameter of 10 µm and a porosity of 80% (produced by Toyo Polymer K.K. and marketed under trademark designation of "RUBYCELL") was trimmed to a thickness of 4 mm and a cross-sectional area of 100 cm² and used to produce a leukocyte separation filter similar to that of Example 1. This filter was incorporated in a circuit configurated as illustrated in Fig. 2. Through this filter, 400 cc of CPD-added concentrated human red cells (CRC) adjusted in advance to a hematocrit value of 50% by addition of physiologicyl saline solution was passed. The time required for this treatment was 6 minutes. By the same analysis as in Example 1, the ratio of removal of leukocytes was found to be 51.7%, that of recovery of red cells to be 95%, and that of removal of platelets to be 42%.

### [Industrial Applicability]

As described above, this invention is directed to a process for separating leukocytes from blood containing leukocytes and erythrocytes, characterised by capturing and adsorbing leukocytes in a filter formed of a porous polyvinyl formal article of a three-dimensionally reticular continuous texture possessing continuous open pores 5 to 30 µm in average diameter. Thus, the filter possesses a high stable ability to capture leukoctyes and is capable of efficiently separating leukocytes from the leukocyte suspension such as the blood or the concentrated red cells. Since this leukocyte separation filter has not use for any filter aid during its operation, it can effect the removal of leukocytes safely without entailing the otherwise possible contamination with foreign matter. It, therefore, permits the red cell fraction for use in the blood component transfusion to be obtained in a highly pure and safe state. This invention contributes in a great measure to such fields of science as medicine and therapy.

Further, the leukocyte separation filter employed in this invention enjoys an outstanding efficiency in the operation thereof for the removal by adsorption of leukocytes when the porous polyvinyl formal article possesses a porosity in the range of 75 to 95% and a wall thickness in the range of 0.5 to 5.0 mm and the continuous open pores possess an average diameter in the range of 8 to 30 µm.

## Claims

1. A process for separating leukocytes from blood containing leukocytes and erythrocytes, characterized by capturing and adsorbing leukocytes in a filter and letting erythrocytes pass therethrough, said filter being made of a polyvinyl formal article having a three-dimensionally reticular continuous texture defining therein continuous open pores of 5 to 30 µm in average diameter.

2. The process according to Claim 1, wherein said porous polyvinyl formal article possesses a porosity in the range of 75 to 95%.

3. The process according to Claim 1 or 2, wherein said porous polyvinyl formal article possesses a thickness in the range of 0.5 to 5.0 mm.

4. The process according to any one of Claims 1 to 3, wherein said continuous open pores possess an average diameter in the range of 8 to 30 µm.

## Patentansprüche

1. Verfahren zur Abtrennung von Leukozyten aus Leukozyten und Erythrozyten enthaltendem Blut, welches dadurch gekennzeichnet ist, daß Leukozyten in einem Filter festgehalten und adsorbiert und Erythrozyten durch den Filter passieren gelassen werden, wobei der Filter aus einem Polyvinylformalformling einer dreidimensionalen netzartigen fortlaufenden Struktur, die darin fortlaufende offene Poren eines durchschnittlichen Durchmessers von 5 - 30 µm festlegt, gefertigt ist.

2. Verfahren nach Anspruch 1, wobei der poröse Polyvinylformalformling eine Porosität im Bereich von 75 - 95% besitzt.

3. Verfahren nach Anspruch 1 oder 2, wobei der poröse Polyvinylformalformling eine Dicke im Bereich von 0,5 - 5,0 mm aufweist.

4. Verfahren nach einem der Ansprüche 1 bis 3, wobei die fortlaufenden offenen Poren einen durchschnittlichen Durchmesser im Bereich von 8 - 30 µm aufweisen.

## Revendications

1. Procédé de séparation des leucocytes à partir de sang contenant des leucocytes et des érythrocytes, caractérisé par la capture et l'adsorption des leucocytes dans un filtre et le passage des érythrocytes à travers ce filtre, ledit filtre étant constitué par un article en polyvinylformal ayant une texture continue réticulaire en trois dimensions délimitant à l'intérieur des pores ouverts continus d'un diamètre moyen de 5 à 30 µm.

2. Procédé selon la revendication 1, dans lequel ledit article en polyvinylformal poreux possède une porosité comprise entre 75 et 95 %.

3. Procédé selon la revendication 1 ou 2, dans lequel ledit article en polyvinylformal poreux possède une épaisseur comprise entre 0,5 et 5,0 mm.

4. Procédé selon l'une quelconque des revendications 1 à 3, dans lequel lesdits pores ouverts continus possèdent un diamètre moyen compris entre 8 et 30 µm.
